# EUROPEAN PATENT APPLICATION

(11) **EP 4 056 118 A1**
(43) Date of publication of application: **14.09.2022**
(21) Application number: 22157666.3
(22) Date of filing: 21.02.2022
(51) Int. Cl.: A61B 5/259, A61B 5/265, A61B 5/266, A61B 5/344

(54) **PATIENT MONITORING ELECTRODES AND ELECTRODE PATCHES**

(30) Priority: 10.03.2021 US 202117197940
(71) Applicant: GE Precision Healthcare LLC, Wauwatosa, WI 53226 (US)
(72) Inventor: SMITH, Robert Brendan, Wauwatosa, 53226 (US); JOHNSON, Christopher Ian, Wauwatosa, 53226 (US)
(74) Representative: Kilburn & Strode LLP

(57) **Abstract**

An electrode configured to adhere to a patient's skin to conduct electrical potentials therefrom includes a substrate, a skin adhesive configured to adhere the electrode to a patient's skin, and a conductor plate mounted on the substrate. A conductive gel is configured to contact the patient's skin and to conduct electrical potentials from the patient's skin to the conductor plate when the electrode is adhered to the patient's skin. A removable separator is positioned between the conductive gel and the conductor plate, wherein the removable separator is configured to be removed prior to operating the electrode to conduct electrical potentials.

## Description

### BACKGROUND

The present disclosure relates to electrodes for patient physiological monitoring, and more particularly to systems and devices to increase shelf life of electrodes.

Surface electrodes, or skin electrodes, are used in patient physiological monitoring for obtaining electrical potentials from the patient's skin. Various types of electrodes are available, including wet electrodes, dry electrodes, active electrodes, and passive electrodes. Active and passive electrodes may each be wet or dry electrodes, and likewise wet and dry electrodes may each be active or passive. Wet electrodes are commonly used because they are easy to apply to a patient and typically yield lower noise levels than dry electrodes. Wet electrodes typically include conductors made of silver/silver chloride (Ag/AgCl) material and include a conductive gel between the patient's skin and the Ag/AgCl conductor. The impedance present between the electrode, and including between the conductor and the patient's skin, is directly related to the amount of noise in the recorded potential, and thus directly relates to the performance of the electrode.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one embodiment, an electrode configured to adhere to a patient's skin to conduct electrical potentials therefrom includes a substrate, a skin adhesive configured to adhere the electrode to a patient's skin, and a conductor plate mounted on the substrate. A conductive gel is configured to contact the patient's skin and to conduct electrical potentials from the patient's skin to the conductor plate when the electrode is adhered to the patient's skin. A removable separator is positioned between the conductive gel and the conductor plate, wherein the removable separator is configured to be removed prior to operating the electrode to conduct electrical potentials.

One embodiment of a fetal ECG patch is configured to be adhered to the skin of a mother's abdomen to record ECG potentials from a fetus and includes a substrate and a plurality of electrodes on the substrate. Each electrode includes a conductor plate mounted on the substrate and a wire connected to the conductor plate and configured to conduct the electrical potentials from the conductor plate. A conductive gel is configured to contact the patient's skin and to conduct electrical potentials from the patient's skin to the conductor plate when the electrode is adhered to the patient's skin. A removable separator is positioned between a conductive gel and a conductor plate. The removable separator isolates the conductive gel from the conductor plate and is configured to be removed prior to operating the electrode to conduct electrical potentials from the patient.

Various other features, objects, and advantages of the invention will be made apparent from the following description taken together with the drawings.

### BRIEF DESCRIPTION OF THE DRAWINGS

The present disclosure is described with reference to the following Figures.
FIG. 1 shows a cross-sectional view of an electrode according to one embodiment of the present disclosure.
FIG. 2 depicts an exploded view of an electrode according to an embodiment of the present disclosure.
FIG. 3 depicts the electrode of FIG. 2 in an assembled form.
FIGS. 4A-4C depict another embodiment of an electrode according to the present disclosure where the removable separator is being removed and then adhered to a patient's skin.
FIG. 5 depicts one embodiment of an electrode patch, and particularly a fetal ECG patch, according to the present disclosure.

### DETAILED DESCRIPTION

The present inventors have recognized problems with wet electrodes, and particularly wet gel electrodes, where the conductive gel causes degradation of the conductive plate on the electrode and thereby shortens the shelf life of the electrode. Namely, the electrolyte gel, such as sodium chloride or potassium chloride, that provides a conductive path from the patient's skin to the conductor plate will cause the Ag/AgCl plate to degrade over time. Electrode performance is mandated by rules and regulations in various jurisdictions, such as by the Food and Drug Administration of the United States government. Electrodes must meet electrical performance characteristics, and testing must be done to verify that a particular electrode manufacture and design meets those electrical performance requirements over time. The degradation caused by the reaction between the conductive gel and the conductive plate is such that many electrode designs do not meet the electrical performance characteristics after a period of time, such as a period of several months or a few years, such as 12-24 months.

The inventors have further recognized that the problem of electrode shelf life is particularly acute for fetal ECG electrodes, which tend to have a shorter shelf life due to a higher concentration of sodium chloride or potassium chloride (or other electrolytic material) in the conductive gel. Fetal ECG electrodes require a lower impedance than regular ECG electrodes or other wet gel patient monitoring electrodes because the measured fetal ECG signal has a much smaller amplitude than a typical adult ECG signal. Thus, the noise level must be lower when recording fetal ECG to maintain a good signal to noise ratio. For instance, a fetal ECG electrode may use a conductive gel having a high sodium chloride concentration of about 10%, compared to a sodium chloride concentration of about 5% for a typical adult wet gel ECG electrode. Similar increases of electrolyte concentrations are provided for other types of electrode gels used for fetal ECG electrode versus standard skin electrodes. The higher electrolyte concentration of in the conductive gel means that the degradation of the Ag/AgCl electrode plate is increased. This reduces the useful shelf life of the fetal ECG electrode compared to a standard wet gel electrode, where a typical shelf life for a fetal ECG electrode may be in the range of 10-12 months.

The current inventors have recognized that the shortened shelf life causes challenges for customers, who must track storage durations of electrodes to ensure that they are used within the shelf life or otherwise disposed of upon expiration. This also causes supply chain problems, where electrode manufacturers and suppliers are under pressure to meet certain deadlines to get electrodes delivered to customers as soon as possible so that the useful lifespan of the electrode is not unduly shortened for the customer.

In view of the problems and challenges in the relevant art, the inventors have developed the disclosed electrode and electrode patch design that includes a removable separator positioned between the conductive gel and the conductor plate. When the removable separator is positioned in the electrode, it isolates the conductive gel from the conductor plate so that no degradation of the conductor plate occurs due to contact with the conductive gel. The removable separator is configured to be removed prior to operating the electrode to conduct electrical potentials from the patient's skin. The removable separator may be configured so that it completely covers the conductor plate and/or any portion of wire or Ag/AgCl material in the electrode that could contact the conductive gel to prevent degradation. The removable separator is then configured to be pulled out or otherwise removed from between the conductive gel and the conductor plate when an operator is ready to use an electrode on a patient. Various embodiments of the removable separator and construction of the electrode for removability of the separator are described herein.

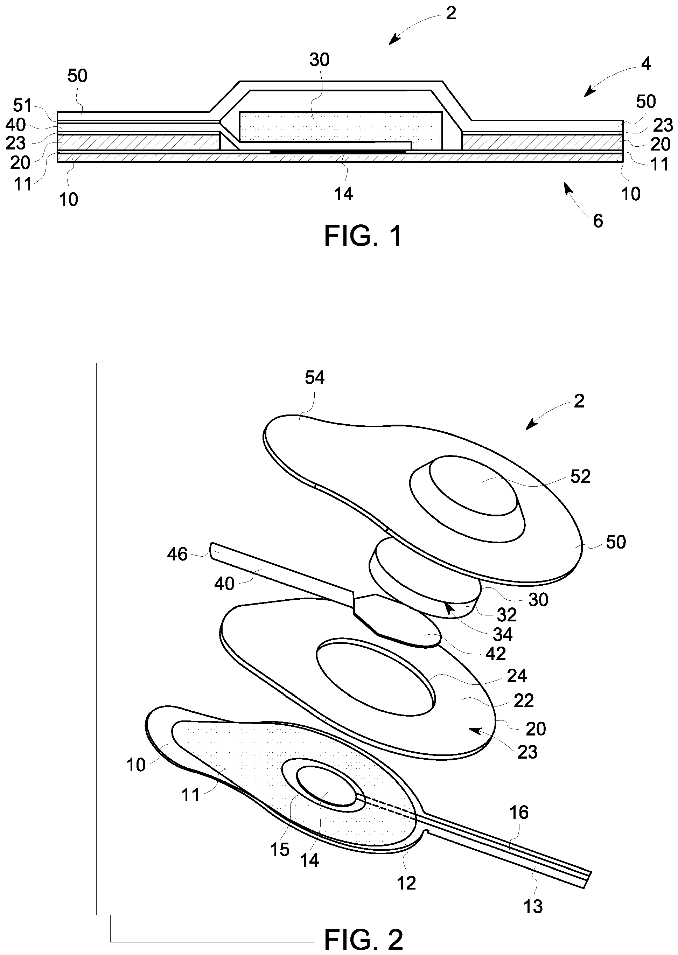

FIG. 1 depicts one example of an electrode 2 having a removable separator 40 positioned between the conductive gel element 30 and the conductor plate 14. The front side 4 of the electrode 2 is configured to adhere to a patient's skin and the substrate 10 forms the back side 6 of the electrode 2. The removable separator 40 gets removed prior to operating the electrode to conduct electrical potentials, such as removal along with or after removal of the release liner 50 on the front side 4 of the electrode 2.

Referring also to FIG. 2, the electrode 2 includes a substrate 10 on which other elements are mounted. For example, the substrate may be a thermal plastic polymer resin, such as polyethylene terephthalate (PET). A conductor is positioned on the substrate, such as conductor plate 14. The conductor plate 14 is connected to the conductive wire 16, which is configured to conduct the electrical potentials absorbed by the conductor plate to a receiver, such as an amplifier circuit, and analog to digital circuit, and/or a patient monitoring device or portion thereof. The substrate 10 may form an electrode portion 12 on which the conductor plate 14 and other electrode components are mounted, and a connector portion 13 on which the wire 16 is mounted, or supported. The conductor, including the conductor plate 14 and the wire 16, may be a conductive metal component that is adhered to the substrate 10, such as comprised of Ag/AgCl. In some embodiments, the conductor plate 14 and the wire 16 may be printed or otherwise deposited onto the substrate 10, such as to form a printed circuit on the substrate 10.

An adhesive layer 11 is mounted on, or applied to, the substrate 10 to mount a skin adhering pad 20 to the substrate 10. The adhesive 11 may be applied in the approximate shape of the skin adhering pad 20 to substantially cover the contact area between the skin adhering pad 20 and the substrate 10. In certain embodiments, the adhesive 11 covers a portion of the wire 16, such as a portion of the wire under the skin adhering pad 20 in the assembled electrode 2. In certain embodiments, the adhesive 11 is applied to avoid the conductor plate 14. In the example at FIG. 2, the adhesive 11 is applied in an oval shape that mimics that of the skin adhering pad 20, where the substrate adhesive 11 has an opening 15 around the conductor plate 14.

The skin adhering pad 20 is configured to be adhered to a patient's skin. For example, the skin adhering pad 20 may be a foam pad 22 with a skin adhesive 23 applied to the front side 4 thereof. For example, a skin adhesive 23 may be 3M 1510 adhesive by 3M Company. In certain examples, the skin adhering pad 20 has an opening 24 with an area at least large enough to accommodate the conductor plate 14 such that, when the electrode 2 is adhered to a patient's skin, electrical potentials are conducted from the patient's skin to the conductor plate 14.

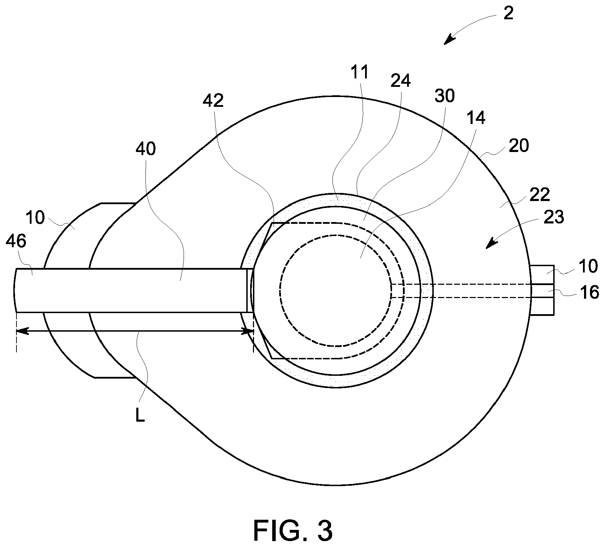

In order to facilitate conduction, electrical potentials from the patient's skin to the conductor plate 14, a conductive gel element 30 is positioned in the opening 24 of the skin adhering pad 20. The conductive gel may be an electrolyte gel of various concentrations, as described above. For example, the conductive gel element 30 may be a sponge 32 with conductive gel 34 suspended therein, such as in the center of the sponge 32. In certain examples, the sponge 32 may be cylindrical in shape having a diameter that is larger than a diameter of the conductor plate 14. When the electrode 2 is assembled and in use, a back side 6 of the conductive gel element contacts the conductor plate 14 and a front side 4 of the gel element 30 contacts the patient's skin. As best seen in FIG. 3, the conductive gel element 30 has a circular surface area that is larger than that of the electrode conductor plate 14, and in the depicted example the circular surface area of the conductive gel element 30 is large enough such that the edge regions of the conductive gel element 30 overlap with the substrate adhesive 11 on the substrate 10.

A release liner 50 may be provided and adhered to a front side 4 of the electrode 2. The release liner is configured to protect the electrode prior to use, including to keep the skin adhesive 23 and the conductive gel element 30 clean and in their original condition to the extent possible. The release liner 50 may have a dome 52 configured to accommodate the conductive gel element 30, which in various embodiments may be proud of the skin adhering pad 20 prior to use of the electrode 2. The release liner 50 may have a shape that is substantially similar to that of the skin adhering pad 20, such as to cover the entirety of the skin adhering pad 20. In certain embodiments, the release liner 50 may extend beyond the skin adhering pad 20 and may include a pull tab 54 configured to be separated from the other electrode components and pulled in order to remove the release liner 50 from the electrode prior to use on a patient. The release liner may be comprised of coated paper material or a plastic material, such as PET.

The electrode 2 includes a removable separator 40 positioned between the conductive gel element 30 and the conductor plate 14 prior to use of the electrode on a patient. The removable separator 40 is configured to be removed from the electrode prior to operating the electrode to conduct potentials from the patient. When the removable separator is in place on the electrode 2, the removable separator isolates the conductive gel in the conductive gel element 30 from the conductor plate 14 so that no corrosion or chemical reaction takes place that can degrade the electrode. This extends the life of the electrode substantially such that degradation of the conductor plate 14 from the conductive gel 34 is not a limiting factor in the shelf life of the electrode 2. Yet, the removable separator can be removed such that the conductive gel 34 immediately contacts the conductor plate 14 and the electrode can operate normally.

The removable separator 40 is removed prior to use of the electrode on the patient, such as prior to applying the electrode 2 to the patient's skin. The removable separator 40 includes a separator portion 42 that is sufficiently large to cover the conductor plate 14. As best shown in FIGS. 1 and 3, the separator 40, and particularly the separator portion 42 thereof, has an area that is larger than that of the conductor plate 14 such that the conductor plate 14 is completely covered and isolated from the conductive gel element 30. The separator portion 42 may overlap onto the substrate adhesive 11.

The removable separator 40 includes a tab portion 46 connected to the separator portion 42 and configured to be pulled to remove the separator portion 42 from between the conductive gel element 30 and the conductor plate 14. The tab portion 46 may have an elongated shape having length L that is sufficiently long such that the tab portion 46 extends out from the conductive gel element 30, and in some embodiments extends out beyond the skin adhering pad 20, and in some embodiments extends outside a boundary of the electrode portion 12 of the substrate 10. In various configurations, the electrode 2 is configured such that the tab portion 46 can be grabbed by a user and pulled to remove the separator portion 42 prior to use of the electrode on the patient.

The removable separator 40 may be formed of plastic material, for example, such as a thin plastic sheet-e.g., comprised of a PET material. In certain embodiments, one or both sides of the removable separator 40 may be coated in a non-stick material, such as silicon. Thus, in various embodiments, the removable separator 40 may be a single-sided siliconized PET sheet, or a double-sided siliconized PET sheet, for example. The silicon coating prevents the removable separator from adhering to the various adhesives on the electrode 2, including the substrate adhesive 11 and the skin adhesive 23 on the skin adhering pad 20. In various embodiments, silicon or other non-stick material may be applied to a front and/or backside of the separator portion 42, and/or applied to a front and/or backside of the tab portion 46 as needed to facilitate appropriate removal of the removable separator 40.

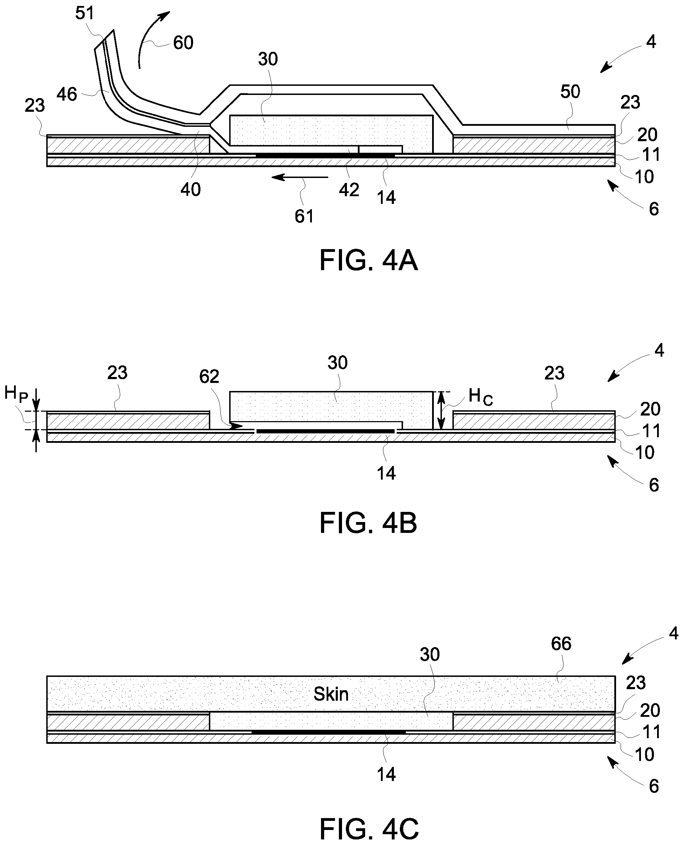

In certain embodiments, the removable separator 40 may be connected to the release liner 50 such that removal of the release liner 50 from the front side 4 also removes the removable separator 40. FIG. 4A depicts such an embodiment. In this example, the tab portion 46 of the removable separator 40 is adhered to the back side 6 of the release liner 50 by an adhesive 51. In such an embodiment, the front side 4 of the tab portion 46 of the removable separator 40 would not be siliconized such that the adhesive 51 adheres to the tab portion 46 sufficiently such that pulling on the release liner 50 will remove the removable separator 40 by pulling the connected tab portion 46. In other embodiments, the removable separator 40 may be connected to the release liner by other means, such as by welding or heat sealing the elements together. In still other embodiments, the removable separator 40 and release liner 50 may be formed as a single, uniform piece.

Referencing FIG. 4A, when the release liner 50 is pulled in the direction of arrow 60, the separator portion 42 of the removable separator 40 is pulled in the direction of arrow 61 in order to remove the separator portion 42 from between the conductive gel element 30 and the conductor plate 14. In this example, the back side 6 of the removable separator 40, including the separator portion 42 and the tab portion 46 may be siliconized to facilitate separation of the removable separator 40 from the substrate adhesive 11 and from the skin adhesive 23. Thereby, the removable separator 40 is easily pulled out when the release liner 50 is removed.

Once removed, an air gap 62 (Fig 4B) may remain between the conductor plate 14 and the conductive gel element 30. However, the conductive gel element 30 is configured to be a compressible element such that when the electrode 2 adhered to the patient's skin 66 the conductive gel element 30 and the conductive gel 34 suspended therein fills any air gap 62 such that complete conductive contact is made between the conductive gel 34 and the conductor plate 14. As shown in FIGS. 4B and 4C, the conductive gel element 30 may be a compressible element that sits proud of the skin adhering pad 20 prior to application of the electrode 2 onto the patient's skin 66. Referring to FIG. 4B, the conductive gel element 30 an initial height Hc that is greater than the height Hp of the skin adhering pad 20. Once applied to the patient's skin 66 as illustrated in FIG. 4C, the conductive gel element 30 is compressed to a height that is substantially the same as the height Hp of the skin adhering pad 20.

In other embodiments, the removable separator 40 may not be adhered to the release liner 50 and thus may be removed from the electrode 2 by a separate step from the step of removing the release liner 50. For example, the tab portion 46 may be configured to visible once the release liner 50 is removed and may be then pulled by a user to remove the separator portion 42 and thereby activate the electrode. In such an embodiment, both sides of the tab portion 46 may be siliconized so that the tab portion 46 does not stick to the release liner 50 and remains in place between the conductor plate 14 and the conductive gel element 30 when the release liner 50 is removed. In other embodiments, the removable separator 40 may be configured to be removed prior to removal of the release liner 50. In such embodiments, tab portion 46 may extend beyond an edge or boundary of the release liner 50 so that it is visible and accessible to a user without removing the release liner 50. Thereby, the user can remove the removable separator 40 to create contact between the conductor plate 14 and the conductive gel element 30 and activate the electrode prior to removal of the release liner 50. In such an embodiment, both sides of the removable separator 40, including the separator portion 42 and the tab portion 46 may be siliconized in order to facilitate removal.

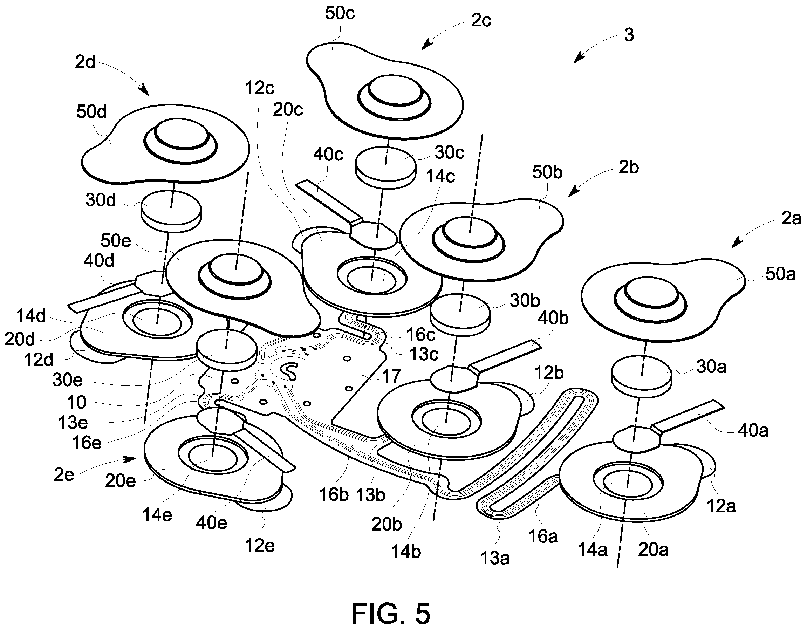

FIG. 5 depicts one embodiment of a fetal ECG patch 3 configured to be adhered to the skin of a mother's abdomen in order to record ECG potentials from a fetus inside the mother's womb. Fetal ECG patches are known in the art, such as the Novii Wireless Patch system by GE Healthcare. As described above, shelf life of fetal ECG electrode patches is a particular problem and concern due to the higher concentration of electrolytes in the conductive gel used in the electrodes in order to provide a lower impedance and better signal to noise ratio. Thus, the removable separator solution disclosed herein may be particularly applicable to fetal ECG electrodes configured for application to a mother's abdomen. However, the disclosed removable separator 40 may be utilized in any wet gel electrode application, such as for standard chest ECG electrodes or other types of skin electrodes, including in other types of electrode patches. For example, the removable separators 40 may be configured in an electrode patch configured for application to a patient's chest for purposes of recording ECG potentials from the patient.

The fetal ECG patch 3 shown in FIG. 5 includes five electrodes; however, patches may include a plurality of electrodes and thus more or fewer than five electrodes may be included in various electrode patch 3 embodiments. The depicted electrode patch 3 examples include a single substrate 10 upon which the electrodes 2a-2e are mounted and formed. The substrate 10 includes five electrode portions 12a-12e, one for each electrode 2a-2e. Each electrode portion 12a-12e of the substrate 10 connects to a connector portion 13a-13e (connector portion 13d is not visible in this exploded view). Each connector portion 13a-13e of the substrate 10 extends from the central portion 17 of the substrate 10 and provides a platform for the wire 16a-16e that connects to each conductor plate 14a-14e. Each electrode portion 12a-12e supports the respective conductor plate 14a-14e, as well as each skin adhering pad 20a-20e and conductive gel element 30a-30e.

Each electrode 2a-2e is provided with a removable separator 40a-40e positioned between the conductive gel element 30a-30e and the respective conductor plate 14a-14e so as to prevent contact between the conductive gel and the conductor plate until activation of each respective electrode. Each electrode also includes a release liner 50 configured to protect the skin adhering pad 20 and the conductive gel element 30. As described above, the removable separators 40a-40e may each be separately removable from the respective electrode 2a-2e such as by pulling on the tab portion 46 thereof, or may be attached to the release liner 50a-50e such that removal of the respective release liner 50a-50e removes the connected removable separator 40a-40e.

This written description uses examples to disclose the invention, including the best mode, and also to enable any person skilled in the art to make and use the invention. Certain terms have been used for brevity, clarity and understanding. No unnecessary limitations are to be inferred therefrom beyond the requirement of the prior art because such terms are used for descriptive purposes only and are intended to be broadly construed. The patentable scope of the invention is defined by the claims, and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

## Claims

1. An electrode configured to adhere to a patient's skin to conduct electrical potentials therefrom, the electrode comprising:
a substrate;
a skin adhesive configured to adhere the electrode to a patient's skin;
a conductor plate mounted on the substrate;
a conductive gel configured to contact the patient's skin and to conduct electrical potentials from the patient's skin to the conductor plate when the electrode is adhered to the patient's skin; and
a removable separator positioned between the conductive gel and the conductor plate, wherein the removable separator is configured to be removed prior to operating the electrode to conduct electrical potentials.

2. The electrode of claim 1, wherein the removable separator isolates the conductive gel from the conductor plate so that no degradation of the conductor plate occurs due to contact with the conductive gel.

3. The electrode of claim 1, wherein the removable separator completely covers the conductor plate.

4. The electrode of claim 3, wherein the removable separator includes a separator portion having an area that is larger than an area of the conductor plate and is configured to completely cover the conductor plate.

5. The electrode of claim 4, wherein the removable separator includes a tab portion connected to the separator portion and configured to be pulled to remove the separator portion from between the conductive gel and the conductor plate.

6. The electrode of claim 1, wherein the conductive gel is suspended in a sponge adhered to the substrate, wherein the conductive gel and the sponge do not contact the conductor plate unless the removable separator is removed.

7. The electrode of claim 1, wherein the removable separator includes a separator portion configured to cover an area of the conductor plate and a tab portion connected to the separator portion and configured to be pulled to remove the separator portion from between the conductive gel and the conductor plate.

8. The electrode of claim 7, wherein the removable separator is configured to be removed from between the conductive gel and the conductor plate prior to adhering the electrode to the patient's skin.

9. The electrode of claim 8, further comprising a release liner on a front side of the electrode connected to the skin adhesive and covering the conductive gel, wherein the removable separator is attached to the release liner such that when the release liner is removed from the front side of the electrode the removable separator is pulled out from between the conductive gel and the conductor plate.

10. The electrode of claim 9, wherein the removable separator has a front side and a back side, wherein at least a portion of the back side contacts the conductor plate and at least a portion of the front side of the removable separator is adhered to the release liner.

11. The electrode of claim 10, wherein the back side of the removable separator is siliconized and the front side of the removable separator is not siliconized.

12. The electrode of claim 7, further comprising a release liner on a front side of the electrode connected to the skin adhesive and covering the conductive gel, wherein the tab portion of the removable separator is covered by the release liner and is configured to be accessed only once the release liner is removed.

13. The electrode of claim 1, wherein the removable separator is a thin plastic sheet having a back side and a front side, wherein at least a portion of the back side contacts the conductor plate and at least a portion of the front side contacts the conductive gel.

14. The electrode of claim 13, wherein at least one of the front side and the back side are siliconized.

15. A fetal ECG patch configured to be adhered to a skin of a mother's abdomen to record ECG potentials of a fetus, the fetal ECG patch comprising:
a substrate;
a plurality of electrodes on the substrate, each electrode comprising:
a conductor plate mounted on the substrate;
a conductive gel configured to contact the patient's skin and to conduct electrical potentials from the patient's skin to the conductor plate when the electrode is adhered to the patient's skin; and
a removable separator positioned between the conductive gel and the conductor plate, wherein the removable separator isolates the conductive gel from the conductor plate is configured to be removed prior to operating the electrode to conduct electrical potentials from the patient.
